# EUROPEAN PATENT SPECIFICATION

(11) **EP 0 661 049 B1**
(45) Date of publication and mention of the grant of the patent: **18.08.1999**
(21) Application number: 93203735.1
(22) Date of filing: 31.12.1993
(51) Int. Cl.: A61K 31/20, A61K 31/23, A23L 1/30, A23L 1/03

(54) **Use of essential fatty acid compositions**
Verwendung von Zusammensetzungen essentieller Fettsäuren
Usage de compositions d'acides gras essentiels

(43) Date of publication of application: 05.07.1995
(73) Proprietor: RIJKSUNIVERSITEIT LIMBURG, 6200 MD Maastricht (NL)
(72) Inventor: Hornstra, Gerard, NL-6228 GR Maastricht (NL)
(74) Representative: de Bruijn, Leendert C.

(56) References cited:
- EP-A- 0 092 076
- EP-A- 0 092 085
- EP-A- 0 181 689
- EP-A- 0 231 904
- EP-A- 0 404 058
- EP-A- 0 440 347
- WO-A-92/12711
- GB-A- 2 197 199
- A.KARLESKIND 'Manuel des corps gras' 1992 , LAVOISIER TEC & DOC , PARIS Volume 1,pages 172-175,264-265 * page 173; table 63 * * page 174; table 66 * * page 264 - page 264; table 21 *

## Description

The present invention relates to the use of essential fatty acids in the preparation of a composition for preventing and/or treating, during and after pregnancy, a deterioration of the essential fatty acid (EFA) status in women.

Compositions containing essential fatty acids (EFA's) and uses thereof are known.

From the German "Offenlegungsschrift" DE-A-3,603,000 an essential fatty acid composition containing highly unsaturated polyene fatty acids from animal and/or vegetable fats is known. This composition can be used for the preparation of foodstuffs for neonates and infants.

European patent application 0,404,058 discloses a similar fatty acid composition containing fatty acids of the (n-3)- and the (n-6)-family. This composition can be used for preparing foodstuffs, especially infant foodstuffs.

From European patent application 0,454,102 essential fatty acid compositions containing a combination of (n-3)- and (n-6)-essential fatty acids for treating schizophrenia and/or associated tardive dyskinesia are known.

European patent application 0,181,689 relates to compositions of copper and linolenic acid, y-linolenic acid and/or dihomo-γ-linolenic acid, which can be used for influencing the 1-series/2-series prostaglandine-balance in the body in favour of 1-series prostaglandins.

Food compositions having an added γ-linolenic acid component for human and animal nutrition are known from U.S. patent 4,938,984.

The international application 92012711 (Martek) describes EFA-containing microbial oils, methods for the preparation of these oils and their use in the preparation of EFA-containing compositions, such as infant formula's and supplements therefor.

WO 92012711 also describes compositions in the form of capsules, of which it is stated that "such capsules provide an easy form of administration to persons having a need for supplementation, such as pregnant of nursing women" and "the composition of the present invention is especially useful as a dietary supplement for pregnant and nursing women".

However, WO 92012711 does not relate to the EFA status of women during and after pregnancy. Also, WO 92012711 does not teach the man skilled in the art that multigravida women will start their second or subsequent pregnancy with an already lower(ed) EFA status.

EP 0 440 347 (Efamol) describes specific gamma-linolenic acid (GLA) and dihomogamma-linolenic acid (DGLA) compositions for increasing, of for preventing a deterioration of, the EFA and/or fat content of breast milk.

As with WO 92012711, EP 0 440 347 does not mention the EFA status of the mother. Also, EP 0 440 347 does not teach or suggest that women who do not breast feed, or who have stopped breast feeding, also need EFA supplementation.

In fact, by suggesting that women who do not nurse their babies or who have stopped nursing their babies do not need EFA supplementation, both WO 92012711 and EP 0 440 347 teach away from the basic recognition underlying the present invention, i.e. that all women need EFA supplementation during and after pregnancy, regardless of whether they nurse their babies or not.

Furthermore, it is known from M.D.M. Al et al, "Biochemical EFA Status of Mothers and their Neonates After Normal Pregnancy", Early Human Development, 24 (1990), 239-248, that the biochemical essential fatty acid status of neonates after a normal pregnancy is not optimal. It is also mentioned in this reference that the neonatal EFA-status depends on the EFA-content of the maternal diet.

A.C. van Houwelingen, J. Puls and G. Hornstra: "Essential fatty acid status during early human developement", Early Human Developement 31 (1992), 97-111, is concerned with the essential fatty acid status of the fetus during the first weeks of gestation.

A.C. van Houwelingen et al: "Essential fatty acid composition of umbilical arteries and veins after fish oil supplementation during pregnancy": in: Drevon, C., Baksaas, I. and Krokan, H.E. (Eds): Omega-3 fatty acids: metabolism and biological effects. Birkhäuser Verlag, Basel, 1993, pp. 125-129, describe that fish oil administration to pregnant women during the last trimester of pregnancy improves the fetal essential fatty acid status.

Furthermore, it is known from Y.T. van de Schouw et al,: "Fatty acid composition of serum lipids of mothers and their babies after normal and hypertensive pregnancies", Prostaglandins Leukotrienes and Essential Fatty Acids 44: 247-252 (1991), that pregnancy induced hypertension (PIH) may lead to a reduction in the linoleic acid status in pregnant women at birth, and also to a mobilisation of the (n-3)-LCP's, especially cervonic acid (22:6 (n-3)) from maternal stores.

However, this reference does not give any information regarding the essential fatty acid status of pregnant women without PIH during pregnancy, or about the essential fatty acid status of women 6 months after a pregnancy, either with or without PIH.

Although from the above prior art some data are available concerning the fetal essential fatty acid status during pregnancy and at birth, none of the cited references gives any information regarding the essential fatty acid status of pregnant women during the course of pregnancy, and/or after pregnancy. In particular, nothing is known about composition of maternal plasma phospholipids (PLs) as measured longitudinally throughout normal pregnancy and after pregnancy.

It has now been found that pregnancy leads to a deterioration of the essential fatty acid status of pregnant women.

It has also been found that this "exhaustion-effect" on the maternal fatty acid-status may result from the mobilization of essential fatty acids from maternal stores, and is not compensated for by normal or increased dietary intake of essential fatty acids during pregnancy.

Furthermore, it has been found that the essential fatty acid-depletion from maternal stores is not completely eliminated after pregnancy by normal dietary uptake, especially where cervonic acid is concerned.

The present invention is therefore concerned with the problem that pregnancy leads to a deterioration of the essential fatty acid status of women during pregnancy and at delivery, which is not compensated for by normal dietary uptake during pregnancy and after pregnancy.

The purpose of the invention is to offer a solution for this problem, which has not been recognized in the prior art.

The present invention therefore relates to the use of one or more essential fatty acids in the preparation of a composition for preventing and/or treating during and/or after pregnancy the deterioration of the essential fatty acid (EFA) status in women.

The essential fatty acids are most preferably chosen from the (n-3)-essential fatty acids and/or the (n-6)-essential fatty acids

As such, the essential fatty acids can be chosen from 18:2 linoleic acid, 18:3 gamma-linolenic acid, 20:3 dihomo gamma-linolenic acid, 20:4 arachidonic acid, 22:4 adrenic acid and 22:5 docosapentaenoic acid or osbond acid (all from the (n-6)-family) and/or 18:3 α-linolenic acid, 18:4 stearidonic acid, 20:4 eicosatetraenoic acid 20:5 eicosapentaenoic acid or timnodonic acid, 22:5 clupanodonic acid, 22:6 docosahexaenoic acid or cervonic acid (all from the (n-3)-family).

It has also been found that the administration to pregnant women of one or more essential fatty acids from the (n-3)-family or (n-6)-family alone leads to a deterioration of the status of the essential fatty acids from the other family in both the mother and the unborn child.

Therefore, according to the present invention a combination of essential fatty acids from the (n-3)- and (n-6)-family is preferably used, more preferably in such amounts, that after administration of the composition the amounts and the ratio of the essential fatty acids from the (n-3)- and (n-6)-families in the body is not unfavourably altered and/or disturbed.

The essential fatty acids can be obtained from animal or vegetal origin, and all suitable sources of (n-3)- and/or (n-6)-essential fatty acids can be used, and such sources are well known in the art. As such, the fatty acids from fish oil of various kinds of fish, from eggs, from cattle and pigs ( especially fat from the liver and the kidneys), vegetable oils, algae, optionally after genetic manipulation, placental tissue etc. can be used, of which the various sources of fatty acids with more than 20 carbon atoms and three or more double bonds are preferred. Also, the usual derivatives of the fatty acids, such as esters, can be used.

The above animal and vegetable oils and fats usually contain a number of essential fatty acids of the (n-3) and/or (n-6)-family, and these mixtures can be used with advantage to prevent deterioration of the (n-3)/(n-6)-essential fatty acid ratio in the body, as such or after further purification, isolation and/or conversion in derivatives.

Also, synthetic essential fatty acids and their derivatives can be used, as well as suitable essential fatty acid compositions from the prior art, such as the compositions from the above mentioned prior art documents.

The composition according to the present invention can be in any suitable pharmaceutical form, and such compositions and the formulation thereof are well known in the pharmaceutical field. As such, the composition can be in the form of tablets, coated tablets, capsules, syrups, elixirs, suspensions, emulsions, solutions, formulations for injection or infusion, etc.

The essential fatty acids are used in the usual amounts, and can be used as such. Furthermore, the composition can optionally contain known additives and further active substances and/or nutritional supplements, such as vitamins and minerals, especially co-factors, such as zinc and its pharmaceutical salts.

The compositions according to the present invention preferably contain one or more antioxidants. Such antioxidants are well known in the art and include for instance citric acid, ascorbic acid, benzoic acid and salts thereof, and the fat-soluble antioxidants, which are preferred, such as tocopherols and tocotrienols etc.

According to the present invention, preferably a composition in the form of a food supplement is used. This supplement can be mixed with the usual diet or taken seperately.

The preparation of food supplements containing essential fatty acids is well known, and can for example be performed as described in EP 0 404 058 and DE-OS-36 03 000 mentioned above or an analogous method.

A daily administration of 0,1-50 g, preferably 1-30 g, more preferably 2-20 g of essential fatty acids is usually effective. However, other amounts can be used, depending on the condition of the subject to be treated and other related factors, as can be determined by the physician of the dietician. The longer-chain, more unsaturated derivatives of linoleic and α-linolenic acids (the long-chain polyenes, LCP's) can probably be used in smaller amounts than the primary essential fatty acids linoleic and α-linolenic acid themselves. The ratio and amounts of the (n-3)- and (n-6)-essential fatty acids in the composition can be determined by the man skilled in the art.

The compositions containing essential fatty acids are used for preventing and/or treating during and after pregnancy the deterioration of the essential fatty acid status in women.

According to one aspect of the invention the essential fatty acid preparations are suitable for the restoration of the essential fatty acid status in the mother after pregnancy, especially lactating women, more particular women who breast feed their babies.

Furthermore, it is known from Y.T. van de Schouw et al mentioned above that pregnancy induced hypertension (PIH) may lead to a reduction in the linoleic acid status in pregnant women at birth, and also to a mobilisation of the (n-3)-LCP's, especially cervonic acid from maternal stores.

According to the investigation which has led to the present invention, after pregnancy this depletion will not be readily restored by the normal dietary uptake, especially where cervonic acid is concerned. Therefore, a further aspect of the present invention relates to the use of essential fatty acids for the preparation of a composition for the restoration of the essential fatty acid status in women after a pregnancy with PIH.

The present invention will be illustrated in the experimental part, together with the figures, in which
figure 1 shows the essential fatty acids of the (n-3)- and (n-6)-families, as well as the non-essential saturated and unsaturated fatty acids of the (n-9)-family, and the biological pathway for the conversion of linoleic acid and α-linolenic acid in Long Chain Polyenes (LCP's).
figure 2 is a graph, showing the maternal EFA-ratio during pregnancy, at delivery (M) and 6 months thereafter (> 0.5 year). U refers to values in cord plasma.
figure 3 is a graph showing the Cervonic Acid Deficiency Index (CADI) during pregnacy, at delivery (M) and 6 months thereafter (> 0.5 year). U refers to values in cord plasma.
figure 4 is a graph showing the difference in cervonic acid status (in % of total fatty acids) between primiparous and multiparous women during pregnacy, at delivery (M) and 6 months thereafter (> 0.5 year). N refers to values in cord plasma.
figure 5 is a graph showing the difference in cervonic acid status (in % of total fatty acids) during gestation and at birth (partus) of women with and without PIH.
figures 6a and 6b are graphs showing the effect of supplementation of pregnant women with linoleic acid on the total amounts of (n-b) fatty acids (sum (n-6)) and (n-3) fatty acids (sum (n-3)) of plasma phospholipids.
figures 7a and 7b are graphs showing the effect of supplementation of pregnant women with linoleic acid on the cervonic acid deficiency (CADI) -index and EFA-ratio of plasma phospholipids.
figures 8a and 8b are graphs showing the effect of supplementation of linoleic acid to pregnant women on the total amounts of (n-b) fatty acids (sum (n-6)) and (n-3) fatty acids (sum (n-3)) of umbilical vessel wall phospholipids.
figures 9a and 9b are graphs showing the effect of fish oil supplementation of pregnant woman on the total amounts of (n-b) fatty acids (sum (n-6)) and (n-3) fatty acids (sum (n-3)) of maternal and neonatal plasma phospholipids.
figures 10a and 10b are graphs showing the effect of fish oil (FO) and control (C) supplementations to pregnant women on the total amounts of (n-b) fatty acids (sum (n-6)) and (n-3) fatty acids (sum (n-3)) of umbilical vessel wall phospholipids.
figures 11 a-d are graphs showing the absolute amounts of various fatty acids bound to phospholipids (mg/L) from maternal venous plasma throughout gestation at delivery (M) and 6 months thereafter (> 0.5 year). U refers to values in cord plasma.
figures 12 a-d are graphs showing the relative amounts (% of total fatty acids) of the fatty acids of different classes bound to phospholipids from maternal venous plasma throughout gestation at delivery (M) and 6 months thereafter (> 0.5 year). U refers to values in cord plasma.
figures 13 a-c are graphs showing the relative (% of total fatty acids) amounts of linoleic acid, arachidonic acid and cervonic acid bound to phospholipids from maternal venous plasma throughout gestation at delivery (M) and 6 months thereafter (> 0.5 year). U refers to values in cord plasma.

Essential fatty acids (EFA) are essential for human health but cannot be synthesized by man. Therefore, they have to be consumed with the diet. EFA serve important structural functions as membrane components. In addition, they are the ultimate precursors of the prostanoids (prostaglandins and thromboxanes), a group of local-hormone-like substances with important bioregulatory functions.

There are 2 families of essential fatty acids, the (n-6) and the (n-3) family. The essentiality of the (n-6) family is recognized for decades already, but that of the (n-3) family has been a matter of debate for some time. However, at present there is no doubt anymore that (n-3) fatty acids are essential for reproductive-, brain- and visual functions.

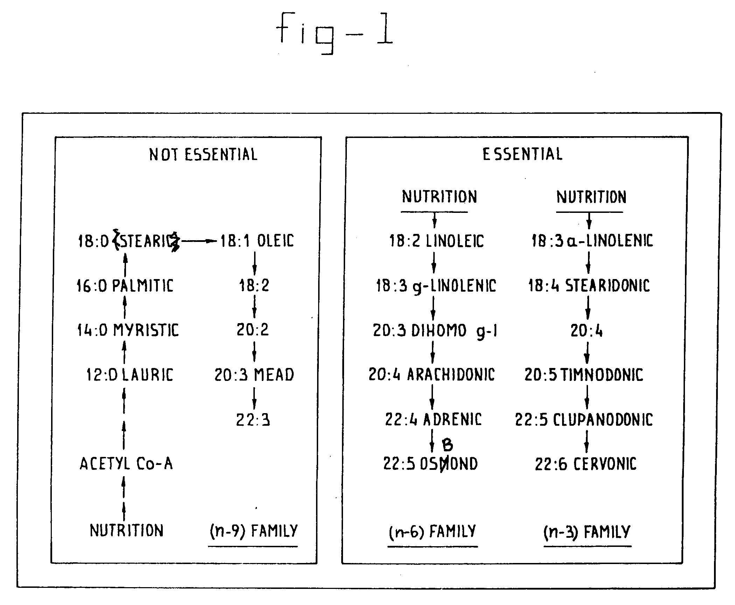

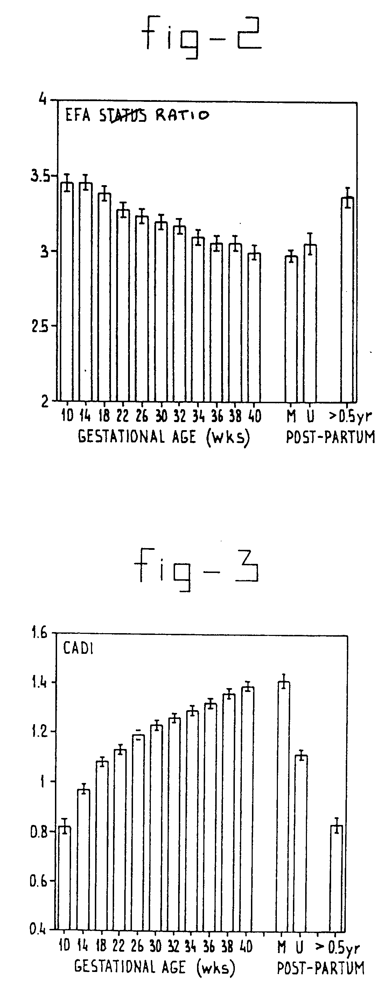

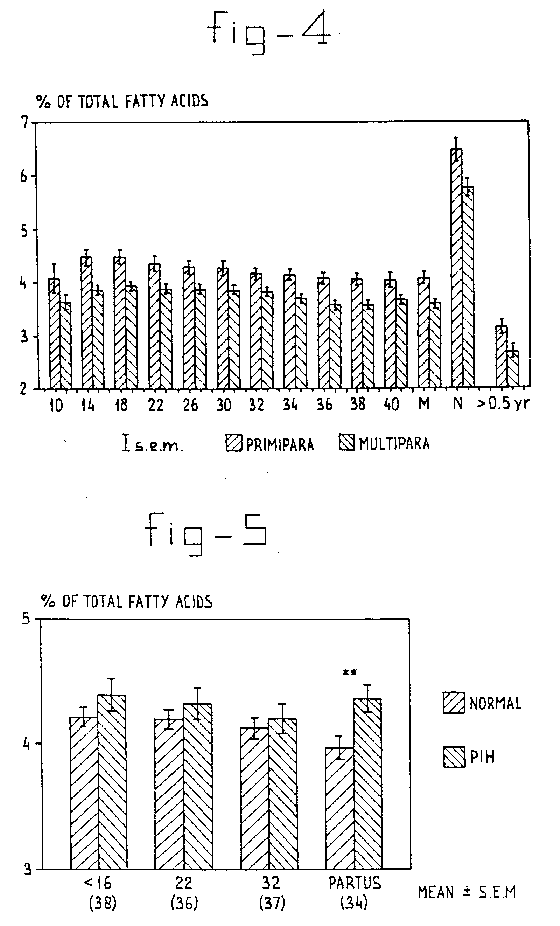

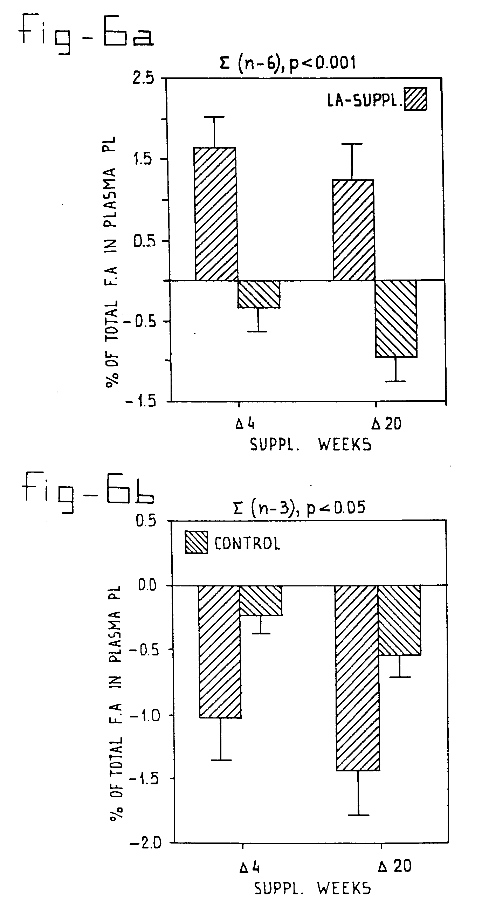

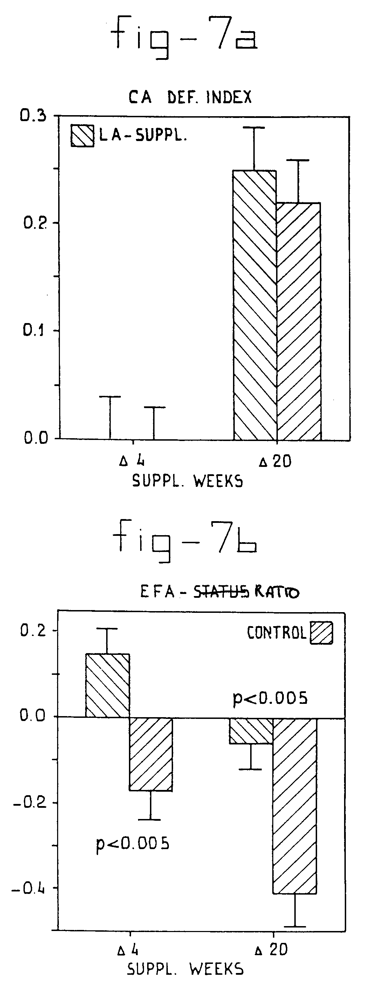

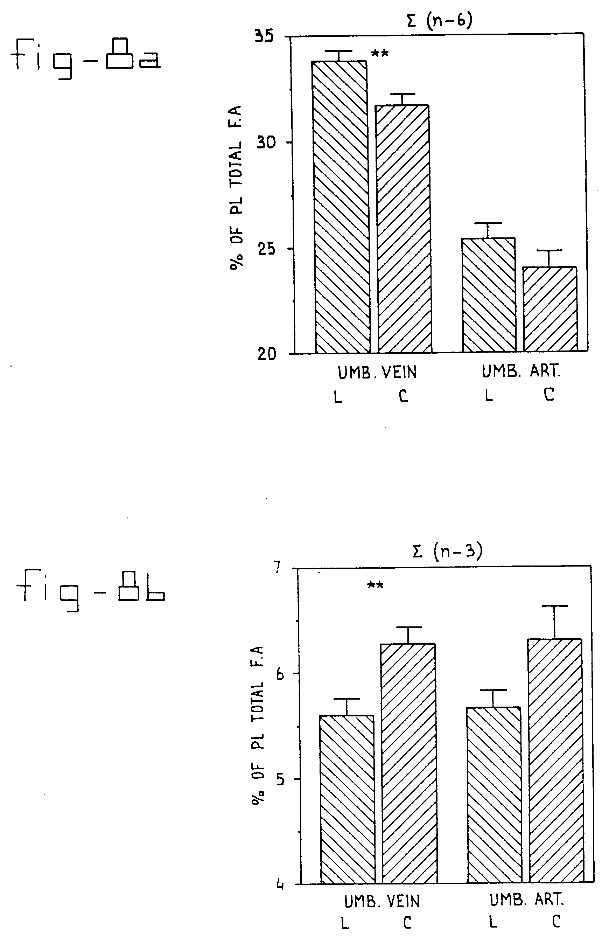

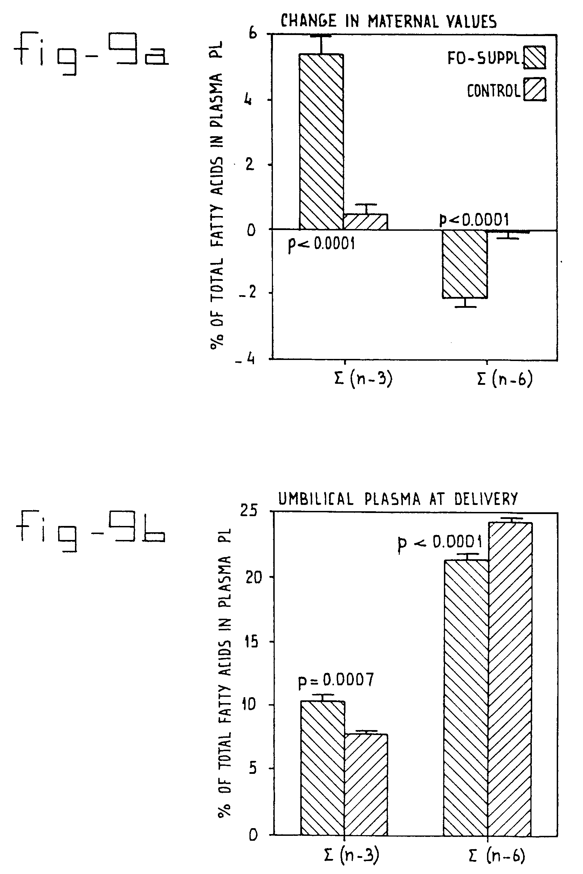

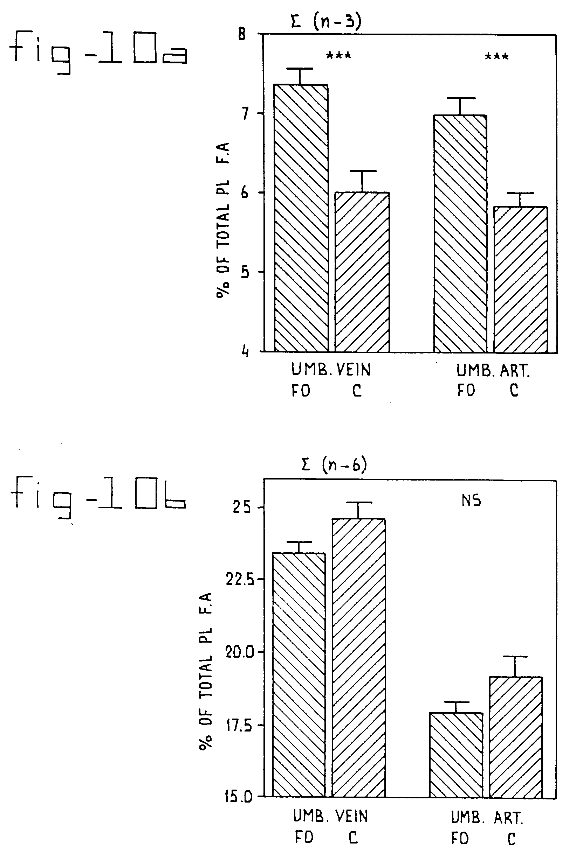

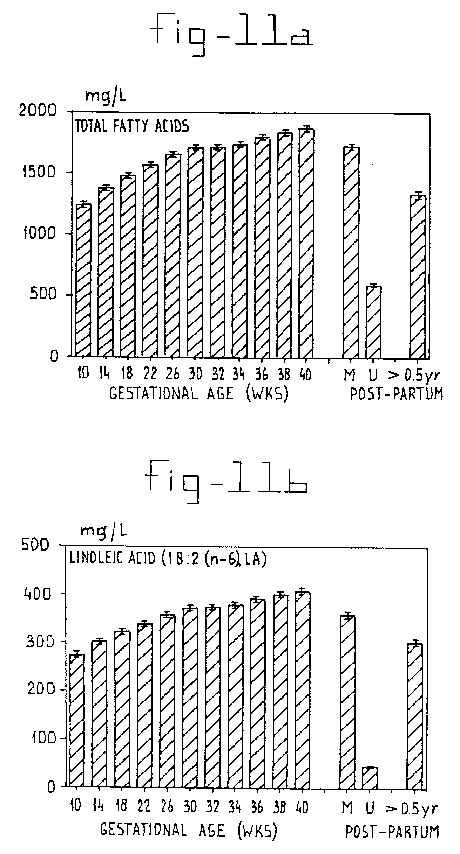

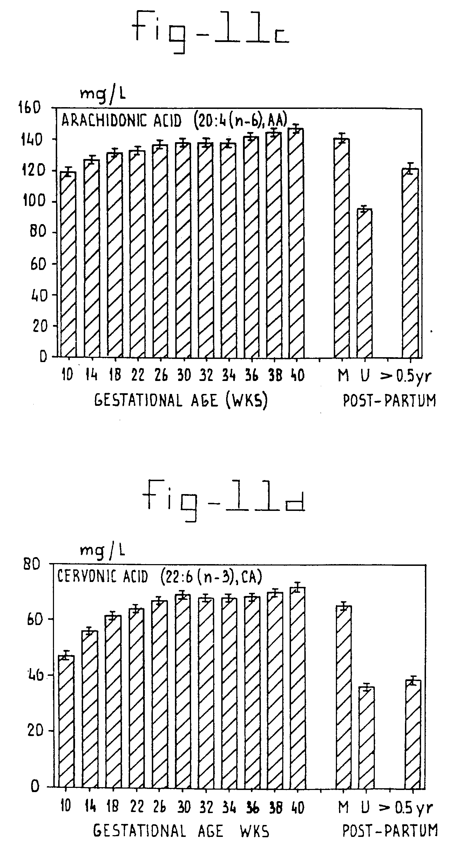

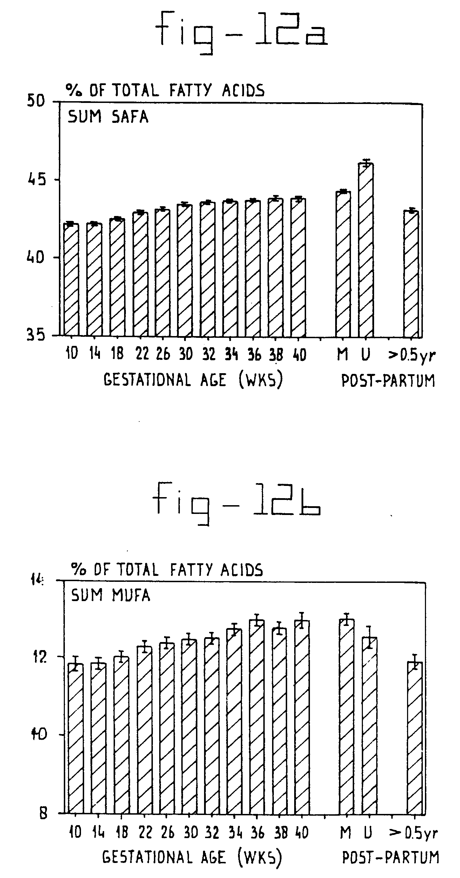

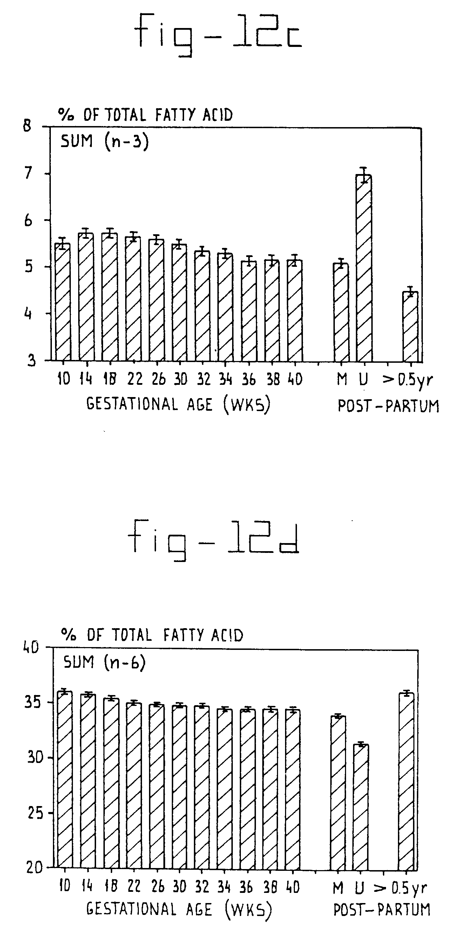

The 'primary EFA' are linoleic acid [18:2(n-6), LA], and alpha-linolenic acid [18:3(n-3) ALA], which are present in seed oils and green leafs. Both primary EFA can be desaturated and elongated in the human body to a series of longer-chain, more unsaturated 'derived EFA'. The (n-3)- and (n-6)-family EFAs and their biological conversion to LCPs are shown in figure 1. The most important EFA derived from LA are dihomo-gamma-linolenic acid [20:3(n-6), DGLA] and arachidonic acid [20:4(n-6), AA]. These fatty acids are the precursors of the prostanoids of the 1- and 2-series, respectively. In addition, AA is an important structural fatty acid in the brain. The most important derived EFA from the (n-3) series are timnodonic acid [eicosapentaenoic acid, 20:5(n-3), TA] and cervonic acid [docosahexaenoic acid, 22:6(n-3), CA]. TA is the precursor of the prostanoids of the 3-series, whereas CA, the major polyenoic fatty acid in brain and retina, has been shown to play an important role in visual and retinal function and in discrimination learning. CA can be converted by a lipoxygenase to form hydroxy fatty acids with biological activity. The other EFA, parent as well as derived, are also good lipoxygenase substrates. Many of the resulting (hydroxy-)fatty acids have significant biological functions also. The derived EFA containing 4 or more double bonds and 20 or more carbon atoms are called the long-chain polyenes (LCP).

Essential fatty acids (EFAs) and their longer-chain, more unsaturated derivatives play a major role during pregnancy. They provide the precursors for prostaglandins and leukotrienes and they are important constituents of all cell membranes, especially those in the brain, and in the nervous and vascular systems. During the third trimester of pregnancy, rapid synthesis of brain tissue occurs and the quantitative accretion in the human brain of the long-chain polyunsaturated fatty acids arachidonic acid (20:4(n-6), AA) and cervonic acid (22:6(n-3), CA) increases steadily with gestation. CA plays an important role in the visual process and the amount of CA in the human retina also increases with maturation. Evidence is growing that CA is essential for a normal development of brain and retina. Therefore, fatty acids like LA, AA and CA may be of great importance in the diet of pregnant women.

Therefore, a longitudinal study to investigate the EFA status of women during and after normal pregnancy was performed. In addition, the relationship between maternal and neonatal EFA status was investigated.

If the dietary intake of EFA is too low to meet the requirement of the body, the EFA body stores will be used as an additional supply. Considerable amounts of linoleic acid are stored in adipose tissue and can readily been mobilized. However, adipose tissue contains hardly any ALA and derived EFA. Since the amount of adipose tissue is rather limited in neonates, in premature infants in particular, an adequate postnatal EFA intake is very important to prevent newborns from becoming EFA deficient.

The developing fetus depends on the mother for an adequate supply of EFA. Therefore, the diet of pregnant women should contain sufficient EFA to cover not only her own requirement, but that of her baby as well. This is not only important during pregnancy, but also after delivery for women who breast feed their neonates. The milk from lactating women contains essential fatty acids -which is one of the main differences between the milk from the mother and most presently available artificial preparations- and a deterioration of the EFA-status of the mother could lead to a non-optimal EFA-content or -composition in the milk, and there is some evidence for this already. This could be a futher reason for EFA-supplementation of women during and after pregnancy.

For the assessment of the EFA status of an individual, the total amount of the various EFA in blood plasma is a useful indicator. Since, in general, phospholipids (PL) have a slower turnover than free fatty acids, triglycerides, and cholesterol esters, the EFA profile of plasma or red blood cell PL is considered suitable to document the overall EFA status of a given individual. It should be realized, however, that the presence of EFA in blood does not necessarily guarantee the proper use of these EFA by cells and tissue. Therefore, functional EFA deficiency markers are required also to properly assess the EFA status of a given individual accurately.

If insufficient EFAs are available to meet the prevailing EFA requirements, the body starts to synthesize 'surrogate fatty acids', which are hardly present under normal conditions. The best known EFA deficiency indicator is Mead acid, 20:3(n-9), MA. This fatty acid is hardly present under normal conditions, but its synthesis is promoted if insufficient parent EFA (LA and ALA) are available to cover the need for the derived EFA. Some of the derived EFA (GLA, AA, CA) can also be taken up directly from the diet, which reduces the need for LA or ALA conversion. Since the derived EFA inhibit MA synthesis, the presence of this latter fatty acid indicates a generalized shortage of all parent and derived EFA.

Another suitable indicator of a generalized EFA shortage is the EFA-ratio, the ratio between the total amounts (%) of all the essential ((n-3) and (n-6)) fatty acids (numerator) and the total amounts (%) of all non-essential (mainly (n-7) and (n-9)) fatty acids (denominator). The higher this ratio, the higher the EFA-status.

Finally, if the body experiences a functional shortage of cervonic acid, it starts to synthesize the most comparable (n-6)LCP, Osbond acid [22:5(n-6), OA]. Therefore, an increased amount of OA indicates a functional shortage of CA. Since the synthesis of OA also depends on the availability of its precursor, adrenic acid [22:4(n-6), ADA], an increased OA/ADA ratio is the most reliable indicator of CA deficiency. Consequently, this ratio is called the Cervonic Acid Deficiency Indicator (CADI). However, under conditions where the (n-6) intake is variable or incomparable, the Cervonic Acid Sufficiency Index (CASI, CA/OA) seems more reliable as a functional indicator of the CA status.

Lipid peroxidation has many adverse effects and, therefore, this process should be carefully controlled. To this end, a large variety of water- and lipid soluble antioxidants are usually available in blood and tissues. Polyunsaturated fatty acids (PUFA) are easily peroxidized and an increased PUFA intake is, therefore, likely to cause more peroxidation in the body. Consequently, the antioxidant requirement is higher, the higher the PUFA consumption.

The parent- and derived essential fatty acids, as well as the LCP are all polyunsaturated fatty acids, and the EFA and LCP supplementations according to the invention may have important consequences for the pre-, peri- and postnatal antioxidant status of mother and child. There are indications that the fetal antioxidant status is rather low. This seems to apply to vitamin E in particular. The changes in blood flow and in oxygenation occurring during birth may have some resemblance to the ischemia-reperfusion phenomenon, which is known to be associated with the generation of large amounts of reactive oxygen species. Uncontrolled lipid peroxidation is considered a major health risk (contributing to cancer, cardiovascular disease, etc.), and fetal and neonatal nutrition is thought to have an important 'imprinting' effect on the disease risks in later life.

Therefore, the EFA supplements according to the invention need to contain sufficient antioxidants to prevent excessive lipid peroxidation. Examples of such antioxidants are citric acid, ascorbic acid and benzoic acid, and the fat-soluble antioxidants, which are preferred, such as tocopherols and tocotrienols.

To obtain longitudinal information on the EFA status of women during the course of normal pregnancy, a venous blood sample was collected from 110 healthy pregnant women at or before 14 weeks of gestation, and every 4 weeks thereafter.

From the 30th week of pregnancy, blood samples were taken bi-weekly. Immediately after delivery and about 6 months later (43 women) blood samples were taken also.

Fatty acid compositions of plasma phospholipids were measured by gas-liquid chromatography and expressed in absolute (mg fatty acid present in phospholipids of 1 liter plasma) as well as relative figures (percentage of total fatty acids in plasma phospholipids).

A continuing increase was observed for the absolute amounts of all essential fatty acids. This is a direct consequence of the increase in plasma phospholipids during pregnancy, and for the LCP it requires either
- an increased dietary intake
- an enhanced conversion of the parent EFA
- an increased mobilization from maternal stores.

No changes were observed in the dietary intake during pregnancy; therefore, the first option is not very likely. Since a considerable part of the dietary linoleic acid is just used for energy production, an enhanced conversion into (n-6)LCP during pregnancy is feasible indeed. However, an increased conversion of alpha-linolenic acid is unlikely to be responsible for the increase of the absolute (n-3)LCP status. In the first place, the dietary intake of alpha-linolenic acid is rather limited and, secondly, the relative abundance of linoleic acid is likely to prevent the desaturation and elongation of alpha-linolenic acid by competition and will, thereby, inhibit the formation of (n-3)LCP. Taken together, the increased absolute amounts of (n-3)LCP observed during pregnancy most probably results from the mobilization from maternal stores, the cell membranes, which may have negative consequences for membrane function.

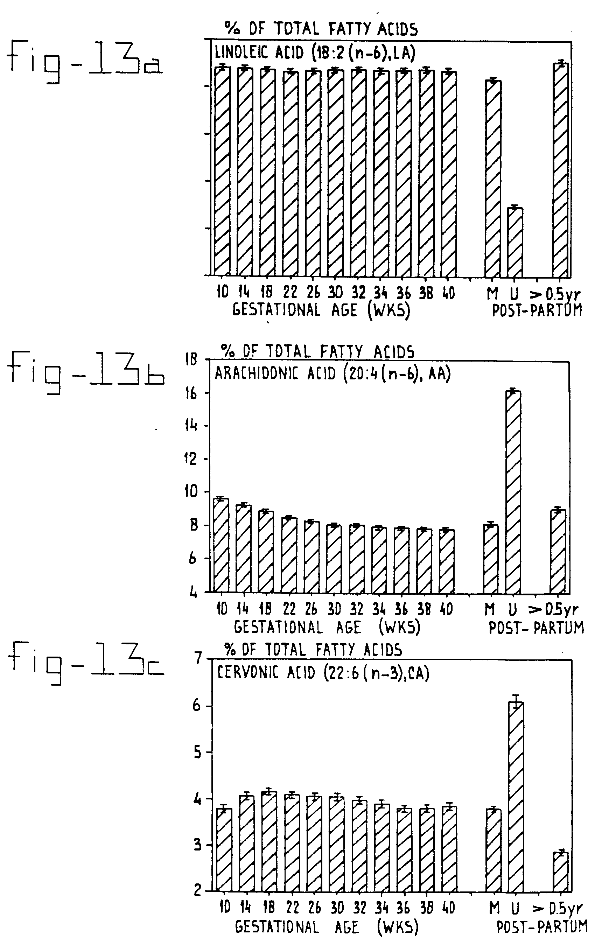

When expressed in a relative way (% of total fatty acids), increases during pregnancy were seen again for the maternal saturated and monounsaturated fatty acids. No change was observed for the relative linoleic acid status, but the relative amounts of (n-6) and (n-3)LCP decreased significantly during pregnancy [for the (n-3)LCP after an initial increase]. This was associated with a decrease of the overall EFA status, can be seen in Figure 2, showing the EFA ratio [ratio between (sum (n-3) + sum (n-6)) and (sum (n-7) + sum (n-9))] during pregnancy (gestational age 10 to 40 weeks, maternal plasma phospholipids), at delivery (M: maternal; U: neonatal) and about 6 month after delivery (maternal only, > 0.5 yr). In addition, the continuous increase of the Cervonic Acid Deficiency Index (CADI) observed during pregnancy indicates an ever continuing deterioration of the cervonic acid status, as can be seen in Figure 3, showing the Cervonic Acid Deficiency Index (CADI) [ratio between 22:5(n-6) (numerator) and 22:4(n-6)] (denominator) during pregnancy (gestational age 10 to 40 weeks, maternal plasma phospholipids), at delivery (M: maternal; U: neonatal) and about 6 month after delivery (maternal only, > 0.5 yr).

This strongly indicates that the LCP-increasing mechanisms are inadequate to meet the higher maternal and fetal LCP demands during pregnancy, and that the EFA content of the maternal diet during pregnancy is inadequate to meet these higher LCP demands. For CA this likely presents a particular problem, since the maternal CA status throughout pregnancy and the neonatal CA status at delivery appeared significantly (10-15%) lower for multigravida as compared to primigravida ( figure 4).

Actually, a significant, negative relationship was observed between parity and the maternal CA status at delivery. This clearly indicates an 'exhaustion-effect' of pregnancy on the maternal CA status. Since, at delivery, highly significant correlations are observed between maternal and cord plasma PL for all EFA, the neonatal CA status can be expected to reduce with birth order. This appeared to be true indeed: as demonstrated in figure 4, which shows the difference in cervonic acid status (% of total fatty acids) between primigravida (n=13-47) and multigravida (n=18-63) women during pregnancy (gestational age 10 to 40 weeks), at delivery (M: maternal; N: neonatal) and about 6 months after delivery (maternal only, > 0.5 yr) -All differences significant (p2 values between 0.038 and <0.001), except at 10 weeks (p2=0.17). In addition, the neonatal CA status is significantly higher in neonates of primigravida women compared to neonates of multigravida women. Consequently, LCP supplementation during pregnancy will not only improve the maternal EFA status but that of the neonate as well, especially in multiparous women.

A further aspect of the invention therefore relates to the use of essential fatty acids in the preparation of compositions for preventing the deterioration of the essential fatty acid status of pregnant multiparous women.

In a prospective, longitudinal investigation, it was confirmed that at delivery, women suffering from PIH show a higher CA-status in plasma PL, as known from Van der Schouw et al, mentioned above, and as can be seen in figure 5, showing the difference in cervonic acid status during gestation (gestational age <16, 22 and 32 weeks) and at delivery (partus) between women who had an uncomplicated pregnancy (normal) and women who developed pregnancy induced hypertension (PIH).

It was also confirmed that, at delivery, the LA status of women who developed PIH is significantly lower than that of women with an uncomplicated pregnancy, as shown by Van der Schouw et al, mentioned above.

The linoleic acid status of normal neonates is very low, a condition that is established very early in pregnancy already (see Puls et al, mentioned above. In addition, PIH is associated with a reduced maternal (n-6) status (see above).

Therefore, it was investigated whether supplementation with linoleic acid during pregnancy does improve the maternal and fetal (n-6) status. A venous blood sample was collected from about 100 pregnant women at their first antenatal medical visit, in order to determine their LA status. If below the P50 value (LA content of plasma PL around 22%), the women entered the study. From the 20th week of pregnancy on, twenty women were given LA-rich products, which resulted in an additional intake of 10.6 g linoleic acid per day, whereas 20 women served as non-supplemented controls. As is illustrated in Figure 6a, LA supplementation caused a significant improvement of the (n-6) status. As can been seen from figure 6b, this was associated with a significant decrease in the (n-3) status as compared to the non supplemented control group. Figures 6a and 6b show the effect of supplementation of pregnant women with linoleic acid (ca. 10 gram/day during the second half of pregnancy) on changes in the EFA percentages of (n-6) and (n-3) fatty acids of their plasma phospholipids. Number of volunteers in supplemented and control group is 22. Σ(n-6/3): total amounts of (n-6) or (n-3) fatty acids. Δ4/20: Changes occurring after 4 or 20 weeks of supplementation.

However, the supplementation had no effect on the Cervonic Acid Deficiency Index, as can be seen from figure 7a. In addition, the EFA ratio, which decreased significantly in the control group during the course of pregnancy, remained rather stable in the supplemented group, resulting in a significantly higher value in the supplemented as compared to the control volunteers (figure 7b). Figures 7a and 7b show the effect of supplementation of pregnant women with linoleic acid (ca. 10 gram/day during the second half of pregnancy) on changes in the Cervonic Acid Deficiency Index (ratio between Osbond acid and adrenic acid) and the EFA ratio (ratio between [Σ(n-3)+Σ(n-6)] and [Σ(n-7+Σ(n-9)]) of their plasma phospholipids, respectively. Number of volunteers in supplemented and control group is 22. Δ4/20: Changes occurring after 4 or 20 weeks of supplementation.

The effect of LA supplementation on the neonatal EFA status was measured in the phospholipids of umbilical plasma and of the walls of umbilical vein (the afferent blood vessel) and arteries (the efferent blood vessels). No significant differences were observed between both groups with respect to the amounts of linoleic acid, and of the EFA deficiency indicators Mead acid and dihomo Mead acid. In plasma an in the umbilical vein, significantly higher amounts of the (n-6) LCPs were found in the supplemented group. Although the trend was comparable for all individual (n-6)LCP, the difference was only significant for 20:3(n-6) in the umbilical vein PL. Comparable supplementation effects were observed in the umbilical arteries, although these effects were not statistically significant.

The higher (n-6) levels in the neonates of supplemented women were associated with significantly lower values for the (n-3)LCP, cervonic acid in particular. This can be seen in figures 8a and 8b, showing the effect of linoleic acid (L) supplementation of pregnant women on the total amounts of (n-6) and of (n-3) fatty acids in of umbilical vessel wall PL. C: non supplemented controls. Σ: total amount of the fatty acids of the family indicated. ** p2<0.01. Although this did not result in higher values for the cervonic acid deficiency index, LA supplementation during pregnancy should preferably be accompanied by supplementation with (n-3)LCP.

The effect of fish oil supplementation to pregnant women on their EFA status and on that of their neonates was studied as follows:
Intervention started at a gestational age of 30 weeks and consisted of the daily supplementation of pregnant women with 2.7 g of fish oil. Control groups received either the same amount of olive oil or nothing. Fish oil administration was associated with a slight (4 days) but significant prolongation of gestation, as is known from Olsen, S.F., Dalby Sørensen, J., Secher, N.J., Hedegaard, M., Brink-Hendriksen, T., Hansen, H.S. and Grant, A.: Randomised controlled trial of effect of fish-oil supplementation on pregnancy duration. Lancet, 339: 1003-1007 (1992). It also resulted in a highly significant (about 80%) increase in the maternal (n-3)LCP status, which was associated with a slight (about 17%), but equally significant reduction of the (n-6)LCP status. Similar but less extensive fatty acid changes were observed in umbilical plasma phospholipids. This can be seen from figures 9a and 9b, showing the effect of fish oil (FO) supplementation during the last 10 weeks of pregnancy on the total amounts of (n-6) and (n-3) fatty acids in of maternal and neonatal (umbilical cord) plasma PL. Number of volunteers: 20 in the FO-supplemented group and 16 in the control group. Σ: total amount of the fatty acids of the family indicated. All differences between the groups: p<0.001.

Changes in the walls of the umbilical veins and arteries were significant for the (n-3)LCP but not for the (n-6)LCP, as can be seen from figures 10a and 10b, showing the effect of fish oil (FO) supplementation of pregnant women on the total amounts of (n-6) and (n-3) fatty acids in of umbilical vessel wall PL. C: non supplemented controls. Σ: total amount of the fatty acids of the family indicated. *** p2<0.001; + p2<0.10; NS: not significant, p2>0.10.

This indicates that fish oil supplementation of pregnant women improves the (n-3)LCP supply to their neonates possibly at the expense of the neonatal (n-6)LCP supply. The (n-3)LCP status of the umbilical artery also increased in the fish oil supplemented group, without changing the (n-6)LCP status. A significant reduction in the CA deficiency marker, Osmond acid, indicated a functional improvement of the neonatal CA status. Fish oil administration significantly improved the general EFA status of the mother during gestation and at delivery; the changes for neonatal plasma and tissue were not significant.

### EXPERIMENTAL PART.

### SUBJECTS, MATERIALS AND METHODS

### Study design

At their first antenatal medical visit, pregnant women were asked to participate in the study. The selection criteria for entering the study were a gestational age less than 16 weeks, singleton pregnancy, Caucasian race, diastolic blood pressure below 90 mm Hg, and the absence of any metabolic, cardiovascular, neurological or renal disorder. In total 140 women entered the study. Excluded were women who developed pregnancy-induced hypertension (n=11) or gestational diabetes (n=2), women who delivered their babies before 37 weeks of gestation (n=8) or after 42 weeks (n=5), and women whose babies were not appropriate for gestational age (<P2.3 (n=3) and >P97.7 (n=1)). Finally, 110 women with an uncomplicated pregnancy met the inclusion criteria. Their relevant clinical information is summarized in table 1. Maternal venous blood samples were collected into EDTA-tubes at 10 weeks of gestation (n=45), at 14 weeks (n=98), at 18 weeks (n=101), at 22 weeks (n=97), at 26 weeks (n=98), at 30 weeks (n=105), at 32 weeks (n=103), at 34 weeks (n=100), at 36 weeks (n=101), at 38 weeks (n=86) and at 40 weeks (n=39). Immediately after delivery, a blood sample from the umbilical vein (n=98) and a maternal venous blood sample (n=95) were collected also. At least six months post-partum, 43 women gave a blood sample once more. The protocol was approved by the Ethics Committee of the University and written consent was obtained from each participant.

**Table 1**

| Characteristics of the study population^{a}. | | | |
|---|---|---|---|
| Age mother (yr) | | 29.5 | 19-43 |
| Gestational age (wks) | | 40.4 | 37-42 |
| Birthweight (g) | | 3403 | 2130-4410 |
| Apgar score (5 min) | | 10 | 6-10 |
| Parity at entry (n) | 0 | 47 | |
| | 1 | 46 | |
| | 2 | 14 | |
| | 3 | 3 | |
| Sex (n) | male | 62 | |
| | female | 48 | |

| | | | |
|---|---|---|---|
| ^{a} Mean and range (minimum-maximum) given, except for parity and sex. | | | |

Two indices were calculated to describe the essential fatty acid status of the mother and her newborn. The cervonic acid deficiency index (CADI), defined as the ratio between 22:5 (n-6) (numerator) and 22:4(n-6), was used because a deficit of 22:6(n-3) (denominator) is accompanied by an increase in the conversion of 22:4(n-6) to 22:5(n-6), resulting in higher CADI values. The other index was the essential fatty acid ratio (EFA ratio), defined as the ratio between the sum of the essential (n-3) and (n-6) fatty acids (numerator) and the sum of the non-essential (n-7) and (n-9) fatty acids (denominator). In general, if the supply of (n-3) and (n-6) fatty acids is sufficient, the desaturation of oleic acid to the long-chain polyunsaturated fatty acids of the (n-9) family is limited, leading to higher EFA ratio values. The EFA ratio was used instead of the classic triene/tetraene ratio (an indicator of an essential fatty acid deficiency), because this ratio does not account for changes in delta-5-desaturase activity.

### Biochemical analyses

Plasma was separated from the erythrocytes by centrifugation and stored at -80° C until fatty acid analysis. Fatty acid composition of the phospholipid fraction was performed using 100 µl plasma samples. A total lipid extract was prepared via a modified Folch extraction ( J.Folch. M.Lees and G.H. Sloane-Stanley, J. Biol. Chem 226:487-509). Before lipid extraction, 50 µl of an internal standard (IS) solution was added. The IS solution consisted of 62 mg 1,2,dinonadecanoyl-*sn*-glycero-3-phosphocholine (PC(19:0)₂), 40 mg 10-heptadecenoic acid (C17:1) to check carry over of free fatty acids during the phospholipid separation procedure (see below), and 100 mg BHT, dissolved in 100 ml MeOH. Aminopropyl bonded silica columns (500 mg) were used to separate phospholipids from the total lipid extract. The phospholipids were saponified and the fatty acids (FA) transmethylated to the corresponding methylesters (FAME) by reaction with 14% BF3 in methanol at 100° C for one hour. The FAMEs were separated and quantified by using a HP 5890 II gas chromatograph, fitted with a 50 m CP Sil5 CB non-polar capillary column with 0.25 mm ID and 0.12 µm film thickness (Chrompack, Middelburg, The Netherlands) and a split ratio of 1:40. The injection temperature was 250° C and the detector temperature was 300° C. The starting temperature of the column was 160° C. After 2 minutes, the temperature increased up to 220° C with a rate of 2° C/min and finally to 280° C with a rate of 6° C/min. The flow rate of the carrier gas (N₂) was 35 ml/min. A standard FAME mixture was used to identify the fatty acid methylesters by means of the retention times. Quantifying the amount of FAMEs was based on the ratio between the peak area 19:0 and the peak area of the fatty acid methylesters in the sample multiplied by the amount of 19:0 present in the internal standard solution. The results were expressed in absolute amounts (mg/L plasma) as well as in relative amounts (% of total fatty acids).

### Statistical analyses

In the gestational period (10 weeks through 40 weeks), not all blood samples were taken at exactly the intended days, for practical reasons. Since we wanted to analyze values at specified gestational ages, the values of the fatty acid amounts and percentages at those days were estimated by lineair interpolation between the days when blood had been sampled. For blood samples taken within one week before or after the intended sampling day, interpolation was considered unnecessary. Because of remaining missing data, means were estimated with adjustment for missing data using the repeated measures analysis program BMDP 5V (Jennrich, R.I., and M.D. Schluchter. 1986. Unbalanced repeated-measures models with structured covariance matrices. *Biometrics* **42**: 805-20 and Dixon, W.J. 1990. BMDP statistical software manual: to accompany the 1990 software release. University of California Press, Berkeley, CA.incorporated here by reference). This program adjusts the means using the correlations between measurement times. Using a model with a quadratic dependence of the fatty acid parameter on time, we tested whether a lineair or quadratic course was present. Data from blood samples at 10 weeks of gestation, immediately post-partum (pp), 6 months after delivery (ad), and from the umbilical vein (uv) were analysed in a similar model. The data from weeks 22-32 were included in this analysis to take full advantage of the missing data adjustment. Comparison of pp, ad, uv, and week 10 was done by the Wald test.

Pearson correlations were used to correlate maternal with neonatal values at delivery and to relate the parameters gestational age, birthweight, head circumference and placental weight and size with maternal values early in pregnancy (week 10, 14 and 18) and maternal and neonatal fatty acid values at delivery. Correlation coefficients were calculated for the CADI and EFA ratio and for the absolute and relative amounts of sumSAFA, sumMUFA, sumn-3, sumn-6, 18:2(n-6), 20:4(n-6) and 22:6(n-3). Multiple regression was performed to adjust for the potential confounding variables parity, maternal height, weight and age, smoking habits and educational level of the mother and sex of the newborn for the relationship between gestational age and fatty acid values. When studying the association between size of the newborn, placental weight and placental size and fatty acid levels, gestational age was also included as a potential confounder.
When groups were compared, Student's t-test was performed.

### RESULTS

Plasma fatty acids of chain lenghts ranging from 14 to 24 carbon atoms were identified; however only the three major essential fatty acids linoleic acid (18:2(n-6), LA), arachidonic acid (20:4(n-6), AA) and cervonic acid (22:6(n-3), CA), the sum of the fatty acids of the n-6 family (sumn-6) and n-3 family (sumn-3), the sum of the saturated fatty acids (sumSAFA) and mono-unsaturated fatty acids (sumMUFA) and the two indices CADI and EFA ratio are discussed. The results are presented in the figures 11-13, showing the mean amount (± s.e.m.) of fatty acids in maternal venous plasma phospholipids (PLs) during gestation, in maternal (M) and umbilical (U) venous plasma PLs immediately after delivery, and in maternal venous plasma PLs at least six months post-partum (> 0.5 year).

### The maternal fatty acid patterns during pregnancy

The average total amount of fatty acids (TF) in maternal venous plasma PLs increased significantly (p < 0. 0001) during pregnancy (figure 11), but the rise in TF became less strong towards the end of gestation (p < 0.0001). Figure 11 shows the absolute amounts of various fatty acids bound to phospholipids (mg/L) from maternal venous plasma throughout gestation, of maternal (M) and umbilical (U) venous plasma at delivery, and of maternal venous plasma six months after delivery (> 0.5 yr). Results are given as mean ± s.e.m. (n = 110, after correction for missing values, see materials and methods).

The mean amount of TF increased by 51% from 1238.11 mg/L at week 10 till 1867.84 mg/L at week 40 of gestation. All the fatty acid families, when expressed as mg/L plasma, showed a similar course: the sumn-6 increased by 44%, the sumn-3 by 41%, the sumSAFA by 57% and the sumMUFA by 65% from week 10 till week 40 (data not shown). When focussed on the individual fatty acids LA, AA and CA (figure 11), the highest increment was for CA (52%), followed by LA (48%) and AA (23%). Six months after delivery, TF in maternal plasma PLs had returned to levels comparable with those at the 14th week of pregnancy. The same applied to sumn-6, sumSAFA, sumMUFA, LA and AA. The mean absolute amounts of CA and sumn-3 had declined to levels even significantly (p < 0.01) lower than at 10 weeks of gestation.

The relative amounts of the fatty acids did show a different pattern as compared to the absolute amounts. For sumSAFA and sumMUFA in maternal plasma PLs, a significant (p < 0.0001) increase was observed throughout pregnancy, as can be seen in figure 12, showing the relative amounts (% of total fatty acids) of fatty acids of different classes bound to phospholipids from maternal venous plasma throughout gestation, of maternal (M) and umbilical (U) venous plasma at delivery, and of maternal venous plasma six months after delivery (> 0.5 yr). Results are given as mean ± s.e.m. (n = 110, after correction for missing values, see materials and methods), in which:
sum SAFA: sum of saturated fatty acids
sum MUFA: sum of monounsaturated fatty acids
sum (n-3): sum of the fatty acids of the (n-3) family
sum (n-6): sum of the fatty acids of the (n-6) family

After a slight increase at the end of the first trimester, the sumn-3 decreased significantly (p < 0.0001) during pregnancy and levelled off at the end of gestation (figure 12c). The mean relative amounts of sumn-6 steadily diminished (p < 0.0001) throughout pregnancy (figure 12d). This latter observation was mainly due to the maternal AA status which declined from 9.6% at week 10 till 7.8% at week 40 (figure 13b). The maternal CA status also diminished, but only after a temporary increase until 18 weeks of gestation (figure 13c). In contrast, the maternal LA status did not change throughout pregnancy (figure 13a).

While six months after delivery the sumn-6 (fig. 12d), ΣMUFA (figure 12b) and the relative amounts of LA (fig. 13a) and AA (fig. 13b) in maternal plasma PL's (figure 13) had returned to early pregnancy levels, the ΣSAFA had levels comparable with those of midway gestation (figure 12). After delivery, the maternal CA status decreased to 2.8% six months post-partum, which is significantly (p < 0.0001) lower than at 10 weeks of gestation, as can be seen in figure 13, showing the relative (% of total fatty acids) amounts of linoleic acid, arachidonic acid and cervonic acid bound to phospholipids from maternal venous plasma throughout gestation, of maternal (M) and umbilical (U) venous plasma at delivery, and of maternal venous plasma six months after delivery (> 0.5 yr). Results are given as mean ± s.e.m. (n = 110, after correction for missing values, see materials and methods). A similar pattern was observed for the sumn-3 (figure 12c) which declined to 4.5% six months post-partum.

The cervonic acid deficiency index (CADI) increased significantly (p < 0.0001) throughout gestation (figure 3) and levelled off towards the end of pregnancy (p < 0.0001). Six months after delivery, the CADI was equal to the CADI at 10 weeks of gestation. The essential fatty acid ratio (EFA ratio) declined continuously throughout the duration of pregnancy. Six months after birth, the maternal EFA ratio had returned to early pregnancy levels (figure 2).

### Comparison between maternal and neonatal fatty acid values

The mean amount of TF in umbilical plasma PLs was substantially lower (p < 0.0001) than all maternal values (figure 11a). This was observed for all four fatty acid families: for sumSAFA and sumMUFA the mean amount (mg/L) in umbilical plasma PLs was only about 35% of that in maternal plasma PLs at delivery; for sumn-6 and sumn-3 these proportions were 32 and 47, respectively (data not shown). The mean amount (mg/L) in umbilical plasma PLs was for LA only 13% of the maternal values at delivery (fig. 11b), while for AA (fig. 11c) and CA (fig. 11d) the percentages were 68% and 56%, respectively (p < 0.0001 for all three fatty acids). The neonatal value for CA was not significantly different from the maternal level six months after delivery (figure 11d).

In contrast to the absolute amounts for AA and CA, the mean relative amounts of AA (fig. 13b) and CA (fig. 13c) in umbilical plasma PLs were significantly (p < 0.0001) higher than all maternal values. However, the mean relative amounts of LA in umbilical venous plasma PLs was significantly (p < 0.0001) lower than that of the mother (figure 13a). This resulted in a significant lower sumn-6 concentration in umbilical plasma PLs (fig. 12d) compared to to maternal plasma PLs, despite the higher neonatal AA levels (figure 13b). The sumn-3 (fig. 12c) and SAFA (fig. 12a) amounts in umbilical plasma PLs were significantly (p < 0.0001) higher than the maternal values. For sumMUFA (fig. 12b) no significant difference between mother and child was observed.

The mean CADI value in umbilical venous plasma PLs was significantly (p < 0.0001) lower than the maternal CADI, immediately after delivery, but significantly (p < 0.0001) higher than the maternal CADI six months post-partum and at 10 weeks of gestation (figure 3).

The neonatal EFA ratio did not differ significantly from the maternal value at delivery, but was significantly (p ≤ 0.005) lower than the EFA ratio in maternal plasma PLs six months after birth and at early pregnancy (figure 2).

In table 2 Pearson correlation coefficients between maternal and umbilical venous plasma values, post-partum, are shown for the total amounts of fatty acids (TF), and for the relative amounts of sumSAFA, sumMUFA, sumn-6, sumn-3, LA, AA and CA. No significant correlation was observed for TF, but all the other fatty acids and fatty acid combinations did show a highly significant positive correlation.

**Table 2**

| Pearson correlation coefficients between maternal and umbilical venous plasma phospholipids fatty acids (n=90). | | |
|---|---|---|
| Fatty acid | r | p |
| TF^{a} (mg/L) | 0.04 | NS |
| ΣSAFA^{b} (% of TF) | 0.45 | < 0.0001 |
| ΣMUFA^{c} (% of TF) | 0.45 | < 0.0001 |
| Σn-6^{d} (% of TF) | 0.60 | < 0.0001 |
| En-3^{e} (% of TF) | 0.60 | < 0.0001 |
| 18:2(n-6) (% of TF) | 0.52 | < 0.0001 |
| 20:4(n-6) (% of TF) | 0.56 | < 0.0001 |
| 22:6(n-3) (% of TF) | 0.60 | < 0.0001 |

| | | |
|---|---|---|
| ^{a} TF: sum of total fatty acids | | |
| ^{b} ΣSAFA: sum of saturated fatty acids | | |
| ^{c} ΣMUFA: sum of monounsaturated fatty acids | | |
| ^{d} Σn-6: sum of fatty acids of the (n-6) family | | |
| ^{e} Σn-3: sum of fatty acids of the (n-3) family | | |

### Relationship of maternal and neonatal fatty acid composition in plasma PLs with some clinical variables and smoking habits of the mother.

Because of the large number of comparisons made, only those with a p ≤ 0.01 are reported. For maternal values, only a significant positive correlation was observed between the amount of AA (% of TF) in maternal plasma PLs at 10 weeks of gestation and birthweight (r = +0.39, p = 0.007, n = 45).

The amount of LA (% of TF) in umbilical plasma PLs was negatively correlated with gestational age (r = -0.37, p = 0.0001, n = 98). The relative as well as the absolute amounts for CA and Σn-3 in umbilical plasma PLs correlated positively with gestational age (% of TF: r = +0.38, p = 0.0001 for CA and Σn-3; mg/L: r = +0.42, p < 0.0001 for CA and r = +0.43, p < 0.0001 for Σn-3). Significant positive correlations were also observed between placental weight and the absolute amounts in umbilical plasma PLs for AA (r = +0.27, p = 0.007), CA (r = +0.29, p = 0.005), Σn-3 (r = +0.29, p = 0.005) and ΣSAFA (r = +0.28, p = 0.006, n = 96). These associations did not change when correcting for the potential confounders parity, maternal height, weight and age, smoking habits and educational level of the mother, sex of the newborn and gestational age.

After adjustment for parity, a significant negative correlation was found between the relative amounts of LA in umbilical plasma PLs and head circumference (partial r = -0.36, p = 0.001). Introducing gestational age in the multiple regression analysis did not alter the above conclusion.

No differences were observed between smokers (n=32) and non-smokers (n=78) for the various fatty acids and fatty acid families in maternal and neonatal plasma PLs at delivery.

The mean absolute amount of CA and Σn-3 in umbilical plasma PL were significantly higher in girls than in boys (CA: 40 ± 2.0 vs 33 ± 1.1, p = 0.009; Σn-3: 46 ± 2.3 vs 38 ± 1.5, p = 0.008). No significant differences in gestational age were observed between boys and girls.

### DISCUSSION

This is the first report about the essential fatty acid composition of maternal plasma phospholipids (PLs), measured longitudinally throughout normal pregnancy, in an absolute (mg/L) and in a relative (% of total fatty acids) manner. The decision to use the PL fraction was based on the fact that PLs are structural lipids, which are the richest source of polyunsaturated fatty acids (PUFA). Moreover, changes in PUFA profile are most pronounced in PLs reflecting the essential fatty acid (EFA) status best.

To correct for the, hardly avoidable, missing values, an analysis model was chosen which estimated the means with adjustment for the missing data. The uncorrected data, however, showed a similar course as the corrected data. Moreover, the correction factor was never more than 2.5%.

The increase in absolute amounts of fatty acids in maternal plasma PLs throughout normal pregnancy is a direct consequence of the enhanced PL concentration during gestation. The total fatty acid increase during pregnancy cannot be calculated, because pre-pregnancy levels are not available. As an approximation, maternal blood samples, taken 6 months after delivery, were analyzed. In these samples, the amounts of most of the fatty acids were comparable with their values at the end of the first trimester. From 18 women information was available at 8 weeks of gestation and the fatty acid levels in the plasma PLs of these samples were lower than at 10 weeks. This strongly indicates that the absolute maternal fatty acid levels measured 6 months after delivery, do not yet reflect true pre-pregnancy levels which are still appreciably lower. During pregnancy, the increment observed for CA in maternal plasma PLs was the highest for all polyunsaturated fatty acids, and compared to pre-pregnancy levels (which are most probably lower than the levels 6 months after delivery - see above) the absolute amounts doubled during gestation. This probably reflects the special requirement for CA by the developing brain and retina.

In theory, the improved absolute maternal CA status can result from a change in dietary habits, an increase in fish consumption in particular. However, no differences in nutrient intake during gestation were observed (Al, M.D.M., et al, Nutrient intake of Dutch women during normal pregnancy with special emphasis on essential fatty acids, mentioned above). Another explanation can be an increase in the activity of the enzymes involved in the synthesis of CA from its precursors. However, this process occurs at a very low rate only and, therefore, it seems more likely that the improved maternal CA status during pregnancy is due to an increased mobilization from maternal stores, although a metabolic re-routing (from energy substrate to structural use) cannot be excluded.

When expressed in relative terms, the maternal CA increase lasted until 18 weeks of gestation. Thereafter a gradual decrease is seen, which may reflect the maternal inability to mobilize adequate amounts for optimal fetal development. This view is supported by the fact that the maternal cervonic acid deficiency index (CADI) increases gradually throughout pregnancy , and that the CADI value in umbilical plasma PLs (although lower than the maternal value at delivery) is significantly higher than the maternal value 6 months after delivery, which is the best available estimate for the value in the non-pregnant state. These observations indicate that the present diet of pregnant women is inadequate to guarantee an optimum CA status of the developing fetus. This may be a rationale for considering CA supplementation during pregnancy.

The AA status (% of total fatty acids) in umbilical venous plasma PL is, just like CA, significantly higher than the maternal relative amounts, despite the decrease of the maternal AA status throughout pregnancy. This might point to a high neonatal need for AA which is the most abundant fatty acid of the (n-6) family in neural tissue and retina.

No changes were observed for the maternal LA status throughout pregnancy. The neonatal relative amounts of LA were almost three times less than the maternal plasma values. Results from a recent study demonstrated that the low fetal amounts of LA originate early in pregnancy. In spite of the low LA levels in umbilical venous plasma PLs, the neonatal EFA ratio did not differ from the maternal EFA ratio at delivery. However, since the neonatal EFA ratio was significantly lower than the maternal EFA ratio six months post-partum and at 10 weeks of gestation, the question arises whether the neonatal EFA ratio is optimal indeed.

The capacity of the placenta and fetal liver to synthesize the long-chain polyunsaturated fatty acids (LCPs) AA and CA from their parent essential fatty acids LA and α-linolenic acid (18:3(n-3), α-LA) respectively is very limited. Therefore, other mechanisms have to be responsible for the different fatty acid profiles for LA, AA and CA between maternal and umbilical plasma PLs. It could be due to preferential transport of LCPs across the placenta, but selective sequestering of AA and CA, at the expense of LA, into phospholipids on the fetal side of the placenta may also be responsible. As a consequence, AA and CA are compartmentalized into a lipid fraction which does not recross the placental barrier and therefore will remain available for the developing tissues, like brain and retina. A possible important role in the transport of LCPs in the fetal circulation may be reserved for α-fetoprotein (AFP). This protein is mainly synthesized by the fetal liver and the yolk sac. Although the biological role of AFP is not completely elucidated, it binds polyunsaturated fatty acids, mainly AA and CA, with high affinity. Because these fatty acids are important components of structural lipids, the transport and cell delivery of LCPs could be a major physiological activity of AFP.

The placenta is, for the greater part, a fetal organ which has a fatty acid composition more similar to that of fetal plasma than of maternal plasma. This might explain why significant correlations were observed for placental weight with some fetal fatty acid values, but not with maternal fatty acid levels.

The present invention was primarily based on data both on the course of the maternal essential fatty acid profiles throughout normal pregnancy and on the EFA status of umbilical venous plasma and maternal plasma, six months after delivery. In addition, the fatty acid composition was determined absolute as mg/L and relative as percentage of total fatty acids. These data could serve as reference values in a further comparison between uncomplicated and complicated pregnancies.

## Claims

1. Use of one or more essential fatty acids in the preparation of a composition for preventing and/or treating the deterioration of the essential fatty acid status in women during and after pregnancy.

2. Use according to claim 1, in which the essential fatty acids are chosen from the family of (n-3)-essential fatty acids and/or the family of the (n-6)-essential fatty acids.

3. Use according to claim 1 or claim 2, in which a combination of the essential fatty acids from the (n-3)-family and the (n-6)-family is used.

4. Use according to claim 3, in which the essential fatty acids from the (n-3)-family and the (n-6)-family are used in such amounts, that after administration of the composition the amounts and the ratio of the essential fatty acids from the (n-3)- and (n-6)-families in the body is not unfavourably altered and/or disturbed.

5. Use according to any of the claims 1-4, in which the composition contains one or more antioxidantia.

6. Use according to any of the claims 1-5, in which the composition is a food supplement.

7. Use according to any of the claims 1-6, in which the composition is suitable for restoring the essential fatty acid status of women after pregnancy, especially lactating women.

8. Use according to claim 7 in which the composition is suitable for restoring the essential fatty acid status of women after pregnancy induced hypertension.

## Patentansprüche

1. Verwendung einer oder mehrerer essentieller Fettsäuren bei der Herstellung einer Zusammensetzung zur Verhinderung und/oder Behandlung der Verschlechterung des Status an essentiellen Fettsäuren bei Frauen während und nach der Schwangerschaft.

2. Verwendung nach Anspruch 1, wobei die essentiellen Fettsäuren aus der Familie der (n-3)-essentiellen Fettsäuren und/oder der Familie der (n-6)-essentiellen Fettsäuren gewählt werden.

3. Verwendung nach Anspruch 1 oder 2, wobei eine Kombination der essentiellen Fettsäuren aus der (n-3)-Familie und der (n-6)-Familie verwendet wird.

4. Verwendung nach Anspruch 3, wobei die essentiellen Fettsäuren aus der (n-3)-Familie und der (n-6)-Familie in solchen Mengen verwendet werden, daß nach Verabreichung der Zusammensetzung die Mengen und das Verhältnis der essentiellen Fettsäuren der (n-3)- und (n-6)-Familie im Körper nicht in ungünstiger Weise verändert und/oder gestört wird.

5. Verwendung nach mindestens einem der Ansprüche 1-4, wobei die Zusammensetzung ein oder mehrere Antioxidationsmittel enthält.

6. Verwendung nach mindestens einem der Ansprüche 1-5, wobei die Zusammensetzung eine Nahrungsmittelergänzung ist.

7. Verwendung nach mindestens einem der Ansprüche 1-6, wobei die Zusammensetzung geeignet ist, den Status essentieller Fettsäuren von Frauen nach der Schwangerschaft, insbesondere von stillenden Frauen, wiederherzustellen.

8. Verwendung nach Anspruch 7, wobei die Zusammensetzung geeignet ist, den Status essentieller Fettsäuren von Frauen nach durch Schwangerschaft induzierter Hypertonie wiederherzustellen.

## Revendications

1. Utilisation d'un ou plusieurs acides gras essentiels pour la préparation d'une composition pour la prévention et/ou le traitement de la détérioration du bilan en acides gras essentiels chez les femmes pendant et après la grossesse.

2. Utilisation selon la revendication 1, dans laquelle les acides gras essentiels sont choisis dans la famille des acides gras essentiels en (n-3) et/ou dans la famille des acides gras essentiels en (n-6).

3. Utilisation selon la revendication 1 ou la revendication 2, dans laquelle on utilise une combinaison des acides gras essentiels de la famille en (n-3) et de la famille en (n-6).

4. Utilisation selon la revendication 3, dans laquelle on utilise les acides gras essentiels de la famille en (n-3) et de la famille en (n-6) en des quantités telles qu'après l'administration de la composition, les quantités et le rapport des acides gras essentiels de la famille en (n-3) aux acides gras essentiels de la famille en (n-6) dans le corps n'est pas modifié de façon désavantageuse et/ou perturbé.

5. Utilisation selon l'une quelconque des revendications 1 à 4, dans laquelle la composition contient un ou plusieurs anti-oxydants.

6. Utilisation selon l'une quelconque des revendications 1 à 5, dans laquelle la composition est un complément alimentaire.

7. Utilisation selon l'une quelconque des revendications 1 à 6, dans laquelle la composition est appropriée au rétablissement du bilan en acides gras essentiels chez les femmes après une grossesse, en particulier chez les femmes allaitant.

8. Utilisation selon la revendication 7, dans laquelle la composition est appropriée au rétablissement du bilan en acides gras essentiels chez les femmes après une hypertension induite par une grossesse.
